**Europäisches Patentamt**

**European Patent Office**

**Office européen des brevets**

(19)

(11) Publication number : **0 465 267 A1**

## EUROPEAN PATENT APPLICATION

(12)

(21) Application number : **91306140.4**

(22) Date of filing : **05.07.91**

(51) Int. Cl.⁵ : **A61M 5/148**

(30) Priority : **06.07.90 JP 178711/90**
**30.08.90 JP 229166/90**
**30.08.90 JP 229167/90**
**21.05.91 JP 116253/91**

(43) Date of publication of application :
**08.01.92 Bulletin 92/02**

(84) Designated Contracting States :
**CH DE FR GB LI**

(71) Applicant : **SEIKO EPSON CORPORATION**
**4-1, Nishishinjuku 2-chome**
**Shinjuku-ku Tokyo (JP)**

(72) Inventor : **Miyazawa, Osamu**
**c/o SEIKO EPSON CORPORATION, 3-5 Owa**
**3-chome**
**Suwa-shi, Nagano-ken (JP)**
Inventor : **Yoshino, Masahito**
**c/o SEIKO EPSON CORPORATION, 3-5 Owa**
**3-chome**
**Suwa-shi, Nagano-ken (JP)**
Inventor : **Ikegami, Toshimasa**
**c/o SEIKO EPSON CORPORATION, 3-5 Owa**
**3-chome**
**Suwa-shi, Nagano-ken (JP)**

(74) Representative : **Miller, Joseph et al**
**J. MILLER & CO. Lincoln House 296-302 High**
**Holborn**
**London WC1V 7JH (GB)**

(54) **Fluid discharge apparatus.**

(57) The present invention provides a fluid discharge apparatus comprising a fluid container (3), means (4) for discharging fluid from the container, drive means (70) for operating the discharge means and a drive circuit (40) for controlling the drive means. The container is contractible, and the discharge means are arranged to engage a portion of the container and by the application of pressure to cause the container to contract for discharging fluid.

EP 0 465 267 A1

FIG. 1

The present invention relates to apparatus for discharging a fluid continuously or intermittently for a pre-set amount of time.

Japanese Laid-Open Patent No. 61-22599 discloses fluid discharge apparatus in which an electric motor is used for a driving force and is coupled by a gear train having a combination of large and small gears to a lever arm, which presses directly against an injection syringe in the form of a piston and cylinder.

However, various problems are inherent in this known fluid discharge apparatus:

The use of such an injector means that the injection cylinder has to be extend in a state in which a fluid is sucked into the syringe, whereby the total length of the syringe needs to be approximately twice the length required for the desired fluid capacity. The overall dimensions of the apparatus are thus extended, which is troublesome particularly when the apparatus has to be carried around.

Packing is used to prevent leakage of the fluid and is subjected to a substantial load at the time when the injection cylinder is pressed in. Furthermore, the variation in pressure may be large.

Also, in the case where a motor which rotates continuously is used for the driving force, a mechanism is needed for feeding back a discharge amount and this renders the construction of the apparatus complex.

In addition, an electro-magnetic actuator is used and so the apparatus is susceptible to electro-magnetic noise and the torque of the actuator may be small so that a reduction gear ratio of the gear train must be increased.

Therefore, it is an object of the present invention at least in its preferred form to provide a compact fluid discharge apparatus which has a small fluid container and in which a small amount of electric current is consumed and the discharge amount can easily be controlled.

According to the present invention, there is provided fluid discharge apparatus comprising a container for storing a fluid, means for discharging fluid from the container, drive means for operating the discharge means, and a drive circuit for controlling the drive means, characterised in that the container is contractible, and the discharge means are arranged to engage a portion of the container and by the application of pressure to cause the container to contract for discharging the fluid.

Since the container is contractible, it is possible to produce a compact apparatus.

Further, by employing drive means in the form of an ultrasonic stepping motor, it is possible to provide an apparatus which is immune to magnetism and in which only a small number of components are required between the drive means and the container for transmitting the driving force to the container.

The present invention will be described further, by way of example, with reference to the accompanying drawings in which:

Figure 1 is a plan view showing an embodiment of a fluid discharge apparatus according to the present invention;

Figure 2 is a side view of the discharge apparatus shown in figure 1;

Figure 3 is a side view of a stepping motor of the discharge apparatus shown in figure 1;

Figure 4 is a sectional view of the stepping motor and a reduction gear train;

Figure 5 is a block diagram of a drive circuit of the discharge apparatus shown in figure 1;

Figure 6 is a timing chart illustrating output signals of a motor driving circuit of the drive circuit shown in figure 5;

Figure 7 is a detailed view of a fluid container of the discharge apparatus shown in figure 1;

Figure 8 is a detailed of a modified fluid container;

Figure 9 is a plan view of a modification of a transmission mechanism of the discharge apparatus shown in figure 1;

Figure 10 is a cross sectional view showing another embodiment of fluid discharge apparatus according to the present invention;

Figure 11 is a block diagram of an ultrasonic stepping motor and a drive circuit for use in a fluid discharge apparatus according to the present invention;

Figure 12 is a cross sectional view of the ultrasonic stepping motor shown in figure 11;

Figures 13(a) to 13(e) are explanatory views showing the ultrasonic stepping motor of figure 11 in operation;

Figure 14 is a plan view of a rotor/stator section of the ultrasonic stepping motor shown in figure 11;

Figure 15 is a plan view showing a modified ultrasonic stepping motor;

Figure 16 is an explanatory view showing step driving of the ultrasonic stepping motor shown in figure 15 in greater detail;

Figure 17 is a phase timing chart corresponding to figure 16;

Figure 18 is an applied voltage timing chart corresponding to figure 16;

Figure 19 is a circuit diagram of a control circuit of the drive circuit shown in figure 11;

Figure 20 is a timing chart for signals generated in the circuitry illustrated in figure 19;

Figure 21 is a timing chart for signals generated in a modified operation of the circuitry illustrated in figure 19;

Figure 22 is a block diagram showing a modification of the drive circuit shown in figure 11; and

Figure 23 is a block diagram showing another modification of the drive circuit shown in figure 11.

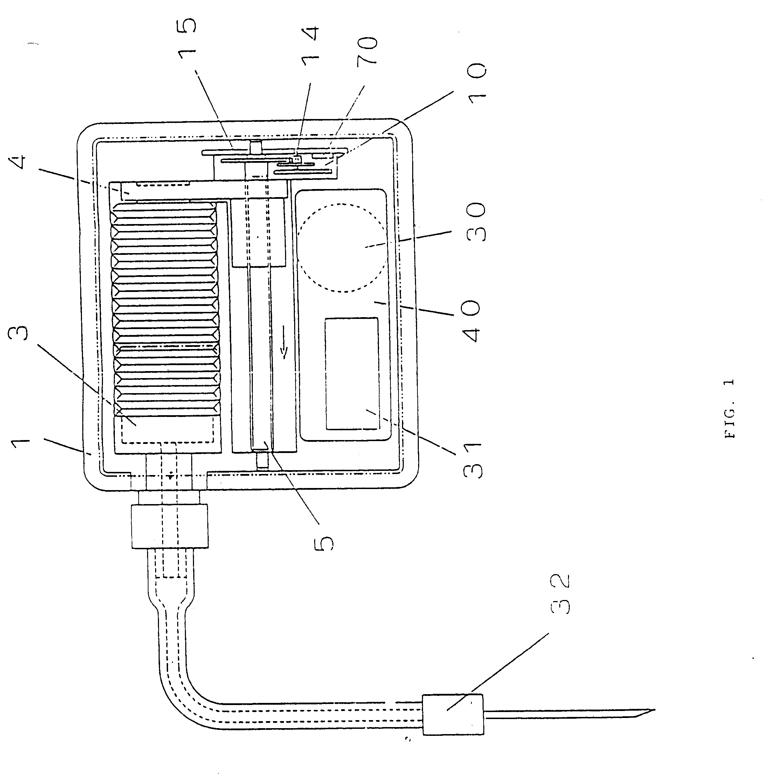

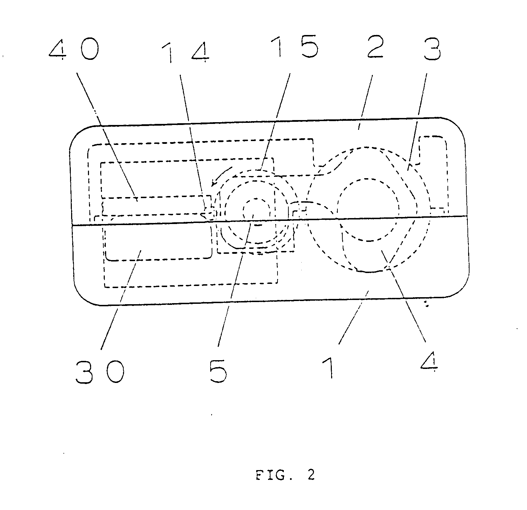

Referring to figures 1 and 2, the discharge apparatus of the present invention comprises a contractible container 3 for a fluid, particularly a chemical fluid, an injection needle including a tube connected to an extreme end of the container 3, a distributed stepping motor 70, and a transmission or transfer mechanism for transmitting the driving force of the stepping motor 70 to the contractible container 3. The transmission mechanism comprises a reduction gear train 10 and a conversion mechanism including a shaft 5 and an actuating lever 4. A circuit 40 drives the stepping motor 70, a button type of electric battery 30 of approximately 1.5V or 3V being employed as a power supply. A display 31 is provided for displaying various conditions of the apparatus, such as discharge amount, an operating state, or an elapsed time period. A upper case 2 is fixed by means of screws or the like to a lower case 1 for providing a casing for locating and protecting the above mentioned respective elements.

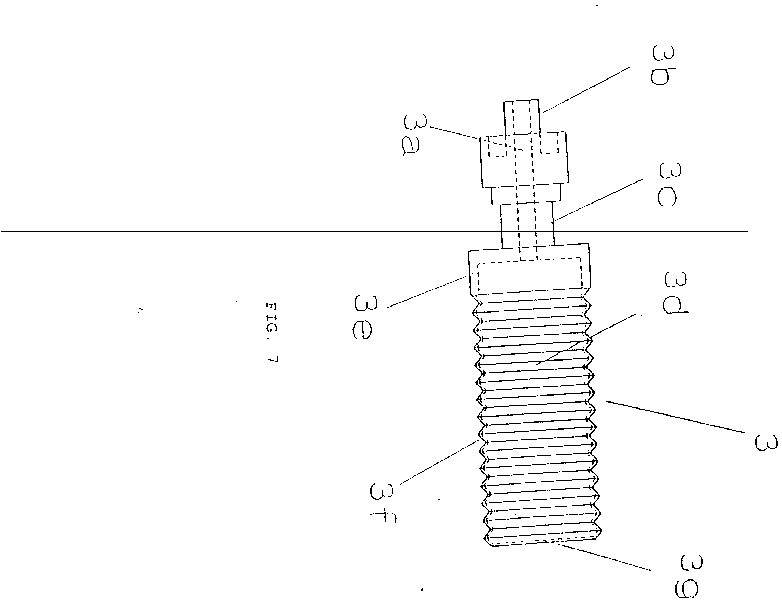

Figure 7 is a detailed view of the contractible container 3 illustrated in figure 1. The container 3 comprises a connection section 3b for direct or indirect connection with the needle or the like, a bore 3a within the section 3b which serves as an outlet or an inlet, a fixture section 3c which is fixed to the apparatus casing, and a storage section 3d for storing the fluid. The storage section 3d has a base section 3e, a bellows section 3f, and a bottom 3g. The thickness of the bellows section 3f is less than the thickness of the base section 3e. In addition, the ratio of the cross sectional area "s" of the inner diameter of the bellows section 3f to the cross sectional area "S" of the outer diameter is set at $s/S \geqq 0.7$ (preferably $s/S \geqq 0.8$). The material of the container may be a synthetic resin, such as polyethlene, vinyl-chloride or the like, and the container may be made by integral moulding. A semi-transparent or transparent material is preferable.

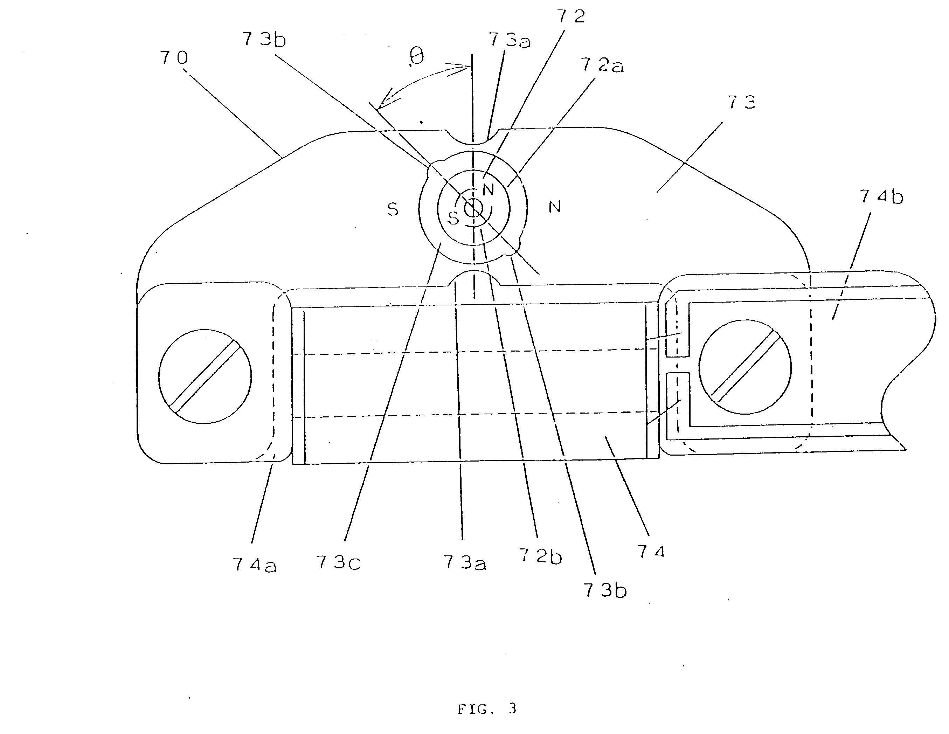

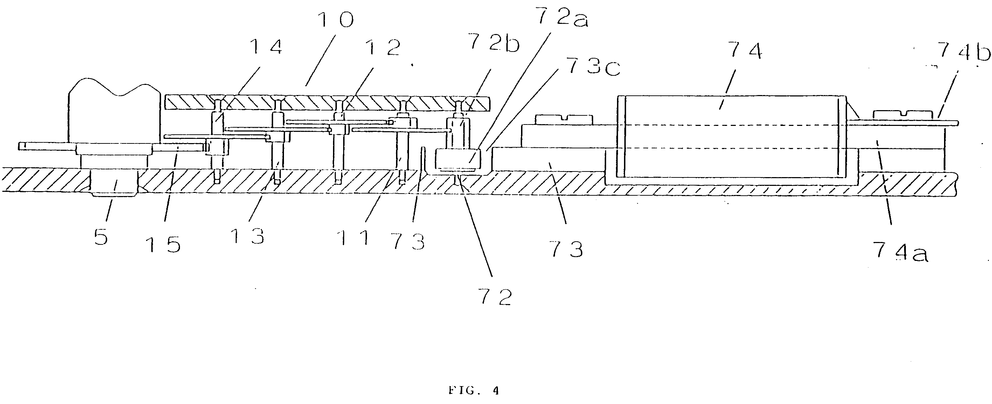

The stepping motor 70 is shown in figures 3 and 4. The stepping motor 70 comprises a rotor 72 consisting of a permanent magnetic 72a (having two poles) and a rotor pinion 72b; a stator 73 having exterior notches 73a, interior notches 73b, and an opening 73c for housing the rotor 72; and a coil block 74 consisting of a lead board 74b to which both ends of a coil wound on a magnetic core 74a are attached. The moment of inertia of the rotor 72 is selected to be 1.5mgmm$^2$ or less; the ampere turns of the coil block are selected to be 3 to 5; and the resistance is selected to be 1.5 to 4.0 K$\Omega$. In the stator 73, the angle $\theta$ between the exterior notch 73a and the interior notch 73b is set to be 38° to 48°. The interior notch 73b has the effect of stopping the magnetic poles N and S of the rotor 72 at a position approximately 90° from the interior notch 73b. A remote end of the lead board 74b is connected to the circuit 40.

By forming the stepping motor in such a manner, it is possible for the stepping motor to consume only a small amount of electric current, eg 2 to 5 µA.

Further, since the coil block and the rotor are separated from each other, a flexible, highly efficient layout is possible. Therefore, the motor can easily be miniaturised.

The reduction gear train 10 comprises the rotor 72b, intermediate wheels 11, 12, 13, and 14, and a gear 15. Its reduction gear ratio is set at 1/14400. The shaft 5 of the conversion mechanism has the gear 15 fixed thereto by interference, caulking, etc, and is threaded for engaging the actuating lever 4, which has a corresponding female screw thread.

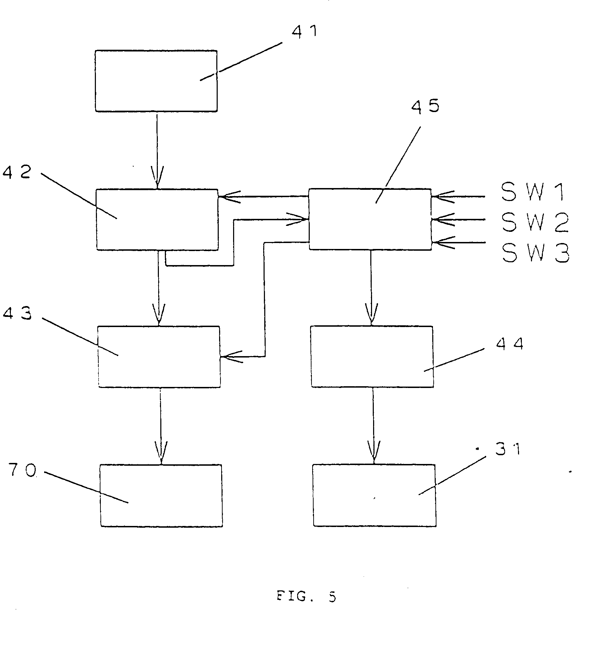

Figure 5 is a block diagram of the drive circuit 40 of the present invention, which comprises an oscillation circuit 41 including a quartz-crystal oscillation element or the like as an oscillation source and a frequency divider 42 for supplying a desired frequency. A motor driving circuit 43 obtains a driving signal for the stepping motor 70. A plurality of switches SW1, SW2 and SW3 serve for setting a desired circuit state, and a control circuit 45 sets the motor driving circuit 43, the frequency divider 42, and a display driving circuit 44 for the display 31 to a desired circuit state accordingly, with signals from the frequency divider 42 and signals from the plurality of switches being used as input signals.

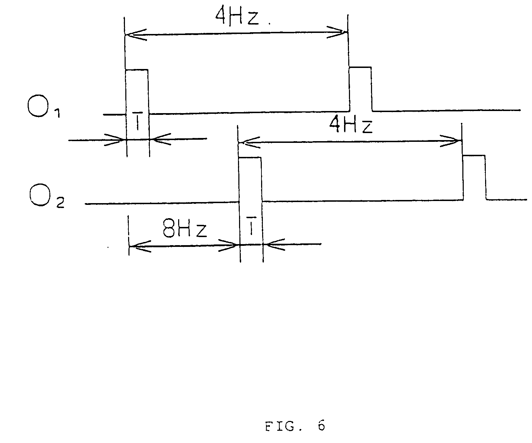

Figure 6 illustrates the driving signals from the above mentioned motor driving circuit 43. A pulse signal $O_1$ is input to one end of the coil and a pulse signal $O_2$ is input to the other end. The signals $O_1$ and $O_2$ both have a frequency of 4Hz, and they have a phase difference which generates a signal with a frequency of 8Hz. The pulse width of each signal $O_1$ and $O_2$ is set between 1.7 and 7.8 ms. The timing and number of pulses are determined by the discharge amount and are not at all limited to the values as specified in the present embodiment.

Next, a description will be provided with the operation of the fluid discharge apparatus.

When the output signal $O_1$ from the motor driving circuit 43 is applied to the coil, a magnetic field is generated. A thin section of the stator 73 formed between the exterior notch 73a and the opening 73c is saturated. As a result, magnetic poles are formed, as shown in figure 3. For this reason, a repulsion force acts on the rotor 72, causing it to first to rotate in a counter-clockwise direction and then to stop rotating. Next, the output $O_2$ is applied to the coil. Because the direction in which the current flows through the coil is opposite to that in the case of the output signal $O_1$, the magnetic poles in the stator 73 are opposite to those shown in figure 3. Thus, the rotor 72 rotates again in a counter-clockwise direction due to the repulsion force.

The rotational force of the rotor 72 is transmitted to the gear 15 via the intermediate wheels 11 to 14 so that the gear 15 rotates in the direction of the arrow in figure 2. Because the shaft 5 rotates in the same direction, the actuating lever 4 is moved in the direction of the arrow in figure 1. The actuating lever 4 thus presses the bottom 3g of the container 3, causing the fluid in the container 3 to be discharged. As the fluid is discharged continuously, the bellows section 3f of the container 3 contracts until the bottom is pressed finally into the position indicated by the dash-dot line in figure 1. The outside diameter of the bellows section 3f is guided by grooves in the lower case 1 and the upper case 2 so that the bellows section contracts reliably in the thrust direction of the actuating lever 4. Therefore, the discharge amount can easily be determined by the number of driving pulses or the like, and the discharge amount need not be detected. As a consequence, a simplified control is possible.

In the present instance, the discharge amount when the container bottom 3g reaches the dash-dot line is approximately 10nl/S, if the average internal diameter of the container 3 is, for example, assumed to be Ø12mm. If the effective capacity of the container 3 is assumed to be 10cc, it can be discharged for about 11 days. A desired discharged amount can be selected by changing one or more of the cross sectional area of the container 3, the frequency of the output signals $O_1$ and $O_2$, the reduction ratio of the gear train 10, the lead angle, pitch and diameter of the screw threads of the shaft 5 and actuating lever 4 etc.

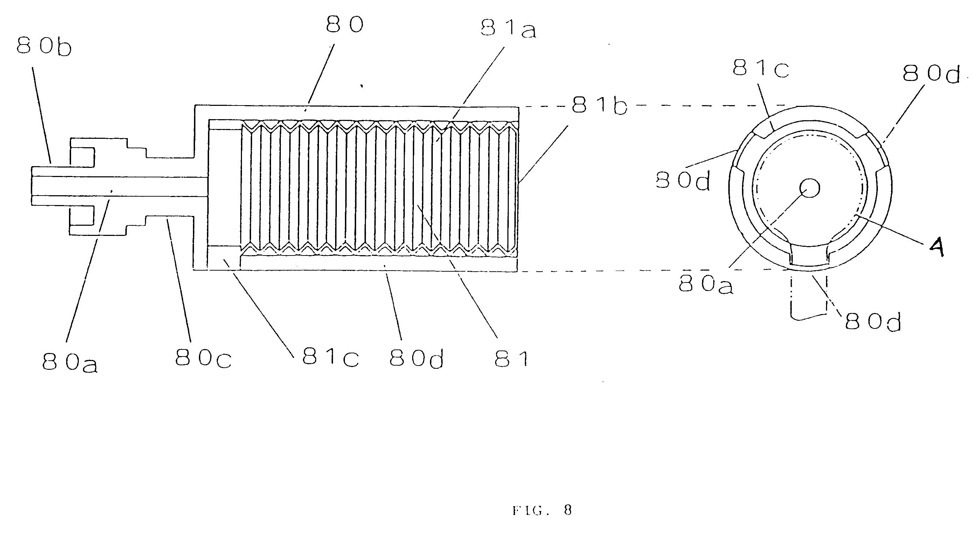

Figure 8 shows another embodiment of a contractible container for use with the apparatus in the present invention. This container comprises an outer frame 80 having a bore 80a within a connection section 80b, a fixation section 80c and three outer notches 80d. The storage section 81 comprises a bellows section 81a, a bottom 81b, and a connection section 81c for connection with the outer frame 80. The outer frame 80 and the storage section 81 are fixed to each other by deposition, bonding, forced fixation or the like so that fluid leakage at the junction will not occur. One of the notches 80d serves for providing access to the actuating lever shown in figure 1 to enable the lever to press against the bottom 81b. The other notches 81d are used for fixing the outer frame 80 and the storage section 81 together. When the bottom 81b is pressed inwardly by the actuating lever at the time of discharge so that the bellows section 81a contracts, there is the possibility that the bellows section 81a might not change its shape smoothly because the bellows section 81a has a thin wall. However, in order to prevent this, the outer diameter of the bellows section 81a is guided by the internal surface of the outer frame 80.

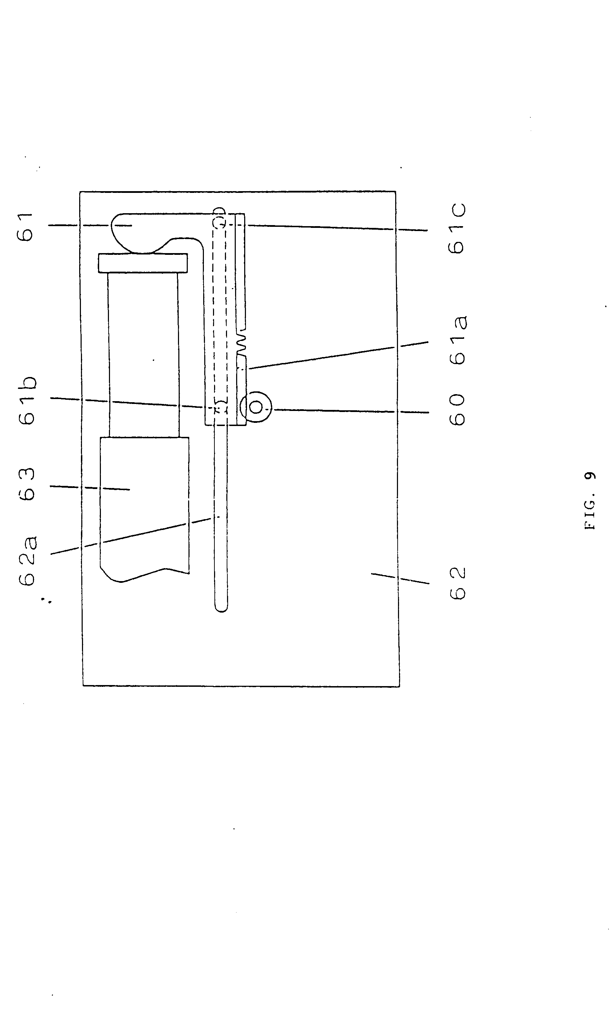

Figure 9 shows a variation on the transmission mechanism described above, which comprises a pinion 60 at the final step of the reduction gear train and an actuating lever 61 having a rack which engages the pinion 60 and converts the rotational motion of the motor to a linear motion. A groove 62a is formed in a lower case 62 or like of the apparatus, and dowels 61b and 61c of the actuating lever are fitted thereto for guiding the lever in its linear motion. Further, the stepping motor and the reduction gear train are placed flush with the apparatus frame 62. With such a construction, the rack can easily be provided by press working or the like even if the attachment of screws is difficult. In addition, the reduction gear train can be placed over the container and the rack, so that the apparatus can be made in a compact planar form.

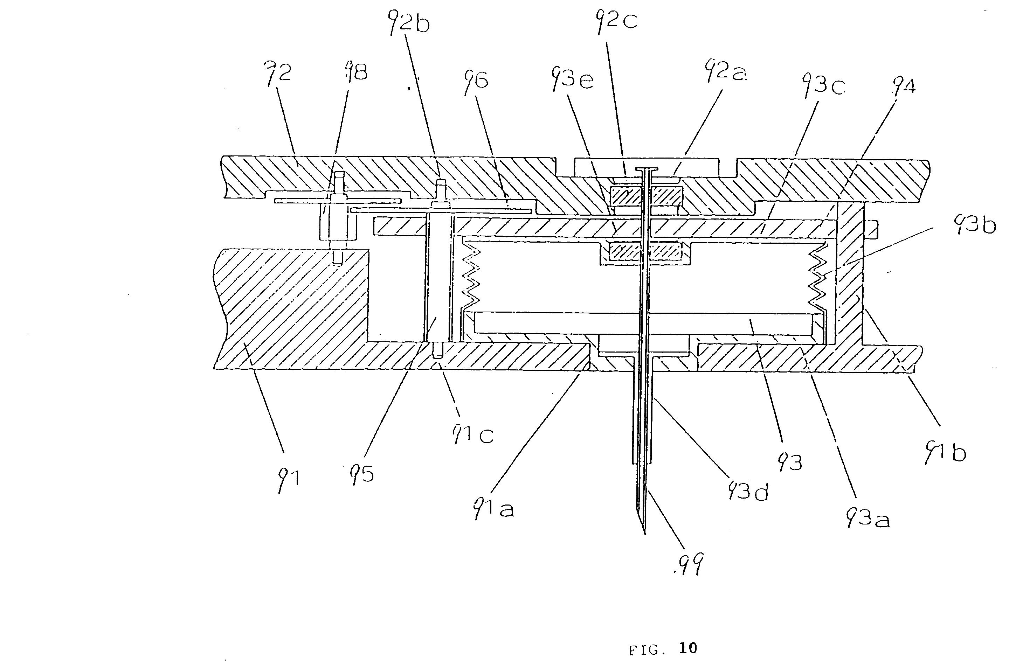

Figure 10 is a partial sectional view showing another embodiment of the discharge apparatus of the present invention. The main points of difference only with respect to the embodiment shown in figure 1 will be explained. The apparatus has a lower case 91 with an opening 91a, with a support shaft 91b and a blind bore 91c, which serves as a bearing for a shaft 95. A container 93 is formed with a base section 93a, a bellows section 93b, an end section 93c, a flexible nozzle 93d, and a lid 93e made of silicon. Each of the sections is bonded to the adjacent sections so as to prevent fluid leakage. An actuating lever 94 having a female screw thread engages a male thread on the shaft 95. A gear 96, which is an interference fit on the shaft 95, engages an intermediate gear 98 forming a part of a reduction gear train for transmitting a driving force from a rotor to the gear 96. An upper case 92 has an opening 92a, and blind bore 92b which serves as a bearing for the shaft 95. A metalic hollow needle is designated by the reference numeral 99.

The main differences with respect to the embodiment shown in figure 1 are that the needle (outlet) is placed perpendicularly to the plane of the case, the container 93 is less elongated, and a driving section not shown is placed in the plane of the case. Also, when the fluid is to be discharged, it is discharged from the bore of the nozzle 93d by withdrawing the needle 99. This opens an opening in the lid 93 but such opening contracts and is instantly closed because the lid 93 is made of a material having a large elasticity. A lid 92c has a similar function for preventing dust, water, or the like from entering.

Next, another embodiment of a drive circuit, for driving an ultrasonic stepping motor serving as an electro-mechanical transducer in a fluid discharge apparatus according to the present invention, will be described.

Figure 11 is a block diagram showing such ultrasonic stepping motor and drive circuit.

In figure 11, a controller 102 supervises the driving timing and the number of driving pulses for the ultrasonic stepping motor. A control circuit 103 receives an oscillating signal from an oscillation circuit 105, having an oscillator which oscillates at a frequency in the vicinity of the resonance frequency of a vibrator 107 of the motor, and a signal from a phase inversion circuit 106 which inverts the phase of the oscillating signal of the circuit 105. The circuit 103 processes these signals, and outputs a control signal to a driver circuit 104, which amplifies the control signal fromthe control circuit 103 and applies a driving voltage to the vibrator 107. Vibration elements of the vibrator 107 are designated 108. In the present example, the vibrator 107 is made up of 4 or more vibration elements 108 as shown and the vibration elements are arranged so that they can vibrate independently of each other.

In figure 11, the output signal of the oscillation circuit 105 is denoted by the reference character ⓐ , and the output signal of the phase inversion circuit 106 is denoted by the reference character ⓑ . The output signal of the controller 102, which output signal serves as a control signal for controlling the output signal ⓐ and the reverse phase signal ⓑ , is denoted by the reference character ⓒ . The output signal of the control circuit 103, which is the driving control signal for driving the vibrator 107 is denoted by the reference character ⓓ .

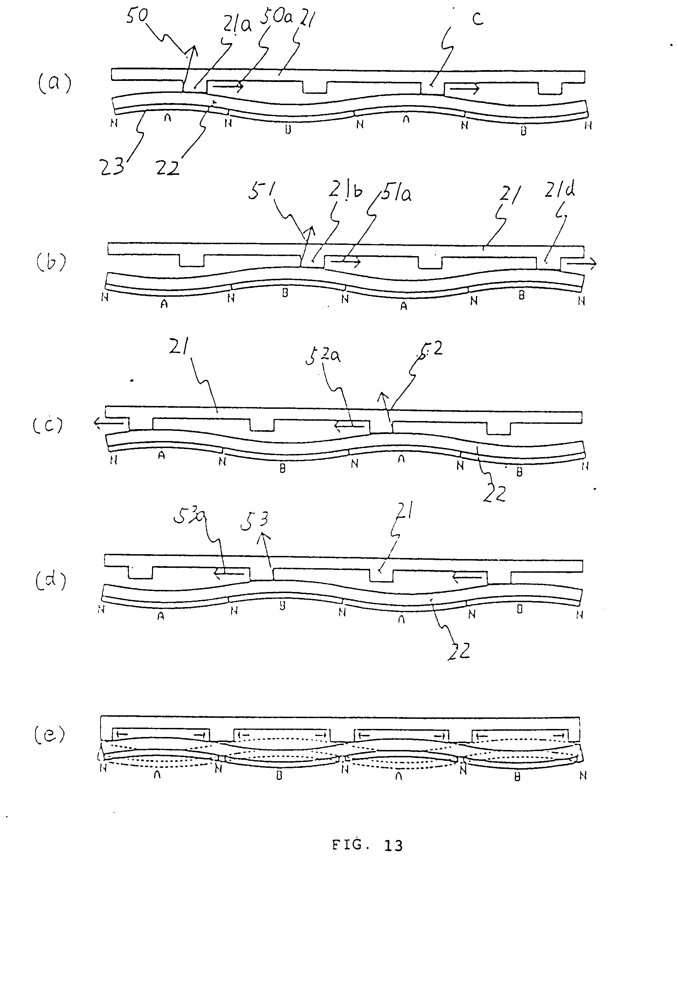

Figures 13(a) to 13(e) illustrate the operation of the ultrasonic stepping motor in a case where a piezoelectric element 23 is used to provide the vibration elements 108.

The stepping motor has a rotor 21 formed with projections 21a, 21b, 21c and 21d. The piezoelectric element 23 is mounted on a stator 22 on a side of the stator opposite to the rotor 21. The piezoelectric element 23 provides the vibration elements, which are classified into two types: namely A and B. The elements A and B are in opposite phase and are deformed relative to each other. A reference character N denotes a node of the vibration mode of the stator 22.

In figure 13(a), the vibration mode of the motor stator is in a pre-determined phase and the projections 21a and 21c are partially in contact with the stator 22. Figure 13(b) shows the stator in a vibration mode having a phase opposite to that shown in figure 13(a), wherein the projections 21b and 21d are partially in contact with the stator 22.

In figure 13(a) and 13(b), the portions of the projections 21a, 21b etc of the rotor 21, which are in contact with the stator 22 and which are positioned at intervals equal to 1 or several times that of the nodes N of the stator 22, are subject respectively to forces 50 and 51 in the directions indicated by arrows. The forces 50 and 51 each have a component 50a and 51a in the direction of the next adjacent node N to the respective projection in that vibration mode. Therefore, the rotor 21 is subject to a force in the direction of the components 50a and 51a, that is a force in the direction of the next nodes N at a position close to the rotor 21.

Figures 13(c) and 13(d) show a case where the positional relationships between the stator 22 and rotor 21 differ from those in figures 13(a) and 13(b) such that the rotor 21 receives force components 52a and 53a (opposite to the components 50a and 51a) derived from forces 52 and 53.

In figure 13(e), the views of figures 13(a) to 13(d) are combined to show that, since in each case the projections 21a to 21d move toward the adjacent node N of the stator 22, the rotor 21 tends to position itself with the projections at the nodes N. Therefore, if the position of each node N moves stepwise, the rotor 21 will move stepwise also.

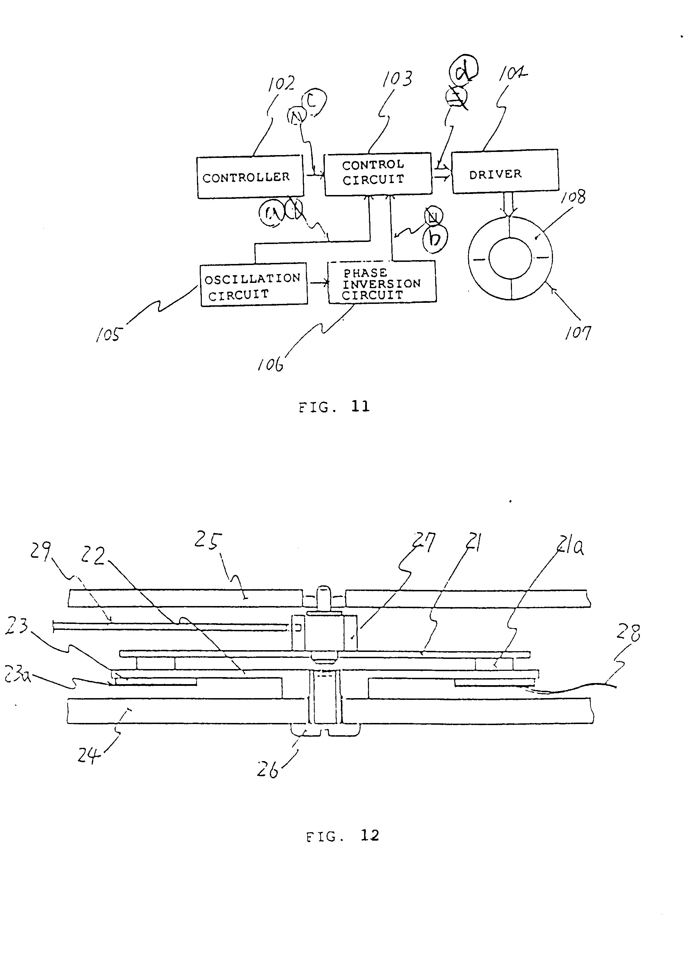

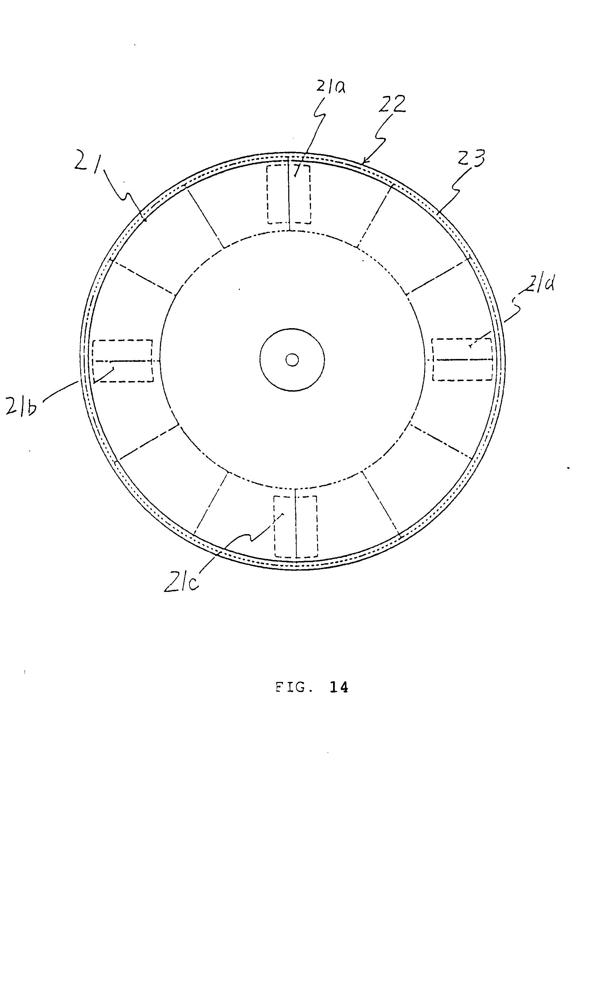

Figure 12 is a cross sectional view showing the construction of the stepping motor of figure 11, and figure 14 is a plan view of the rotor/stator arrangement of the motor. As shown in these figures, the stator 22 is fixed to a ground plate 24 by a screw 26, and a gear train receiver 25 serves for rotatably guiding the rotor 21. A pinion 27 transmits the rotational force of the rotor 21 to the reduction gear train via an intermediate gear 29. A voltage is applied to an electrode pattern 23a of one of the piezoelectric elements by means of a lead wire 28. In this embodiment, the projections 21a to 21d extend axially of the rotor 21.

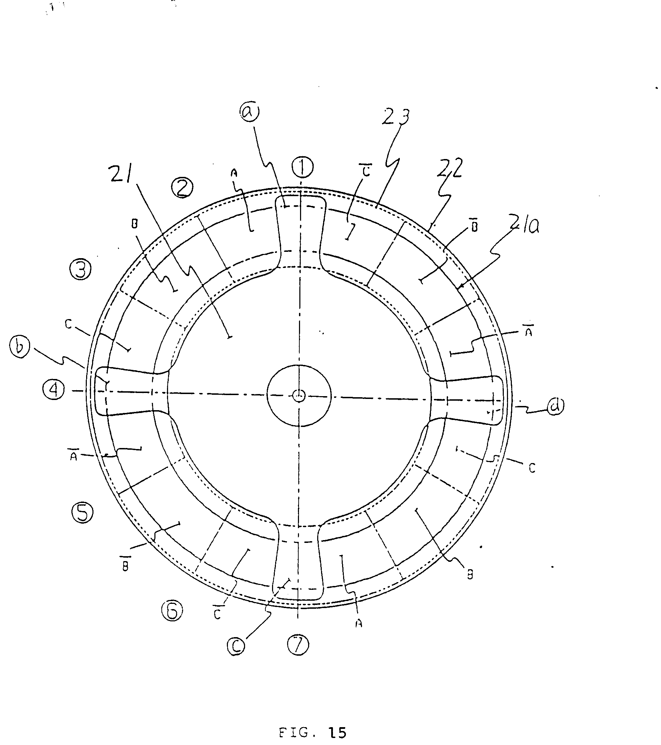

In a modification of the above arrangment shown in figure 15, the piezoelectric element 23 extends around the entire circumference of the stator 22, and the projections 21a to 21d of the rotor 21 project in a radial direction.

In the arrangements of both figures 14 and 15, the rotor 21 and the stator 22 are brought into contact with each other in four places. The piezoelectric element 23 is made up of 12 vibration elements, as shown by dashed lines, which will be designated by the symbols A, B, C, Ā, B̄ and C̄ by way of explanation. A driving voltage in phase is applied to those vibration elements designated by the same symbols. Symbols ① to ⑦ are indicated in sequence in positions where nodes of a corresponding vibration mode occur. In the present embodiment, nodes N are actually created at four places, there being 12 places around the entire circumference at which nodes can be created.

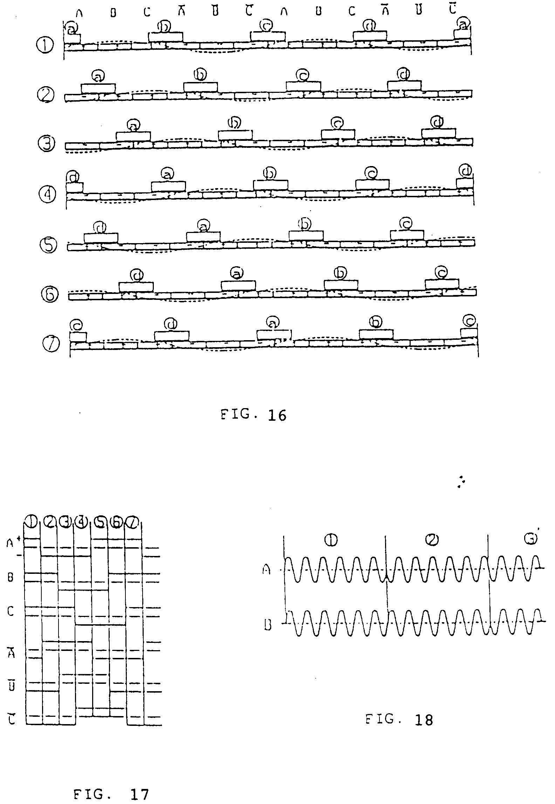

Figure 16 is an explanatory view showing the driving operation of the above mentioned ultrasonic stepping motor, wherein symbols corresponding with those in figure 15 are employed and the ultrasonic stepping motor is presented as linear for the sake of explanation. The dashed lines indicate the form of the vibration mode. The phase of a voltage applied to each vibration element at a given moment is indicated by the symbols + and - for convenience.

In the vibration mode ① , the vibration elements A, B and C and the vibration elements Ā, B̄ and C̄ each constitute a set, and the elements oscillate with each set delayed relative to the previous set by a phase difference of 180°. Therefore, the projections 21a, 21b, 21c and 21d are positioned as shown. In the vibration mode ② , the elements B, C and Ā, and the elements B̄, C̄ and A each constitute a set. In the vibration mode ③ , the elements C, Ā and B and the elements C̄ , A and B each constitute a set. The vibration modes ④ to ⑥ repeat the sequence, and the rotor 21 moves stepwise. In the case of the example shown in figure 15 the rotor 21 makes 1 rotation each 12 steps.

If the vibration modes① to⑥ are performed in a reverse order, for example when switching is performed so as to obtain the vibration mode② subsequent to the vibration mode③ , the rotor 21 moves stepwise in the direction reverse to that obtained in the above mentioned case.

Figure 17 is a timing chart representing the phases of the voltages for producing the vibration modes shown in figure 16. For example, when the vibration mode① is to be produced, a driving voltage of a positive (+) phase is applied to the elements A, B and C, and a driving voltage of a negative (-) phase is applied to the elements $\overline{A}$, $\overline{B}$ and $\overline{C}$ under the control of the control circuit 103. If the output signal ⓐ from the oscillation circuit 105 is assumed to have a positive (+) phase, the output signal ⓑ of opposite phase from the circuit 106 is set to have a negative (-) phase. Switching is performed at a timing determined by the control signal ⓒ output by the controller 102.

Figure 18 is a timing chart representing the driving voltages applied to the vibration elements A and B. For example, in the vibration mode② , the phase of the driving voltage applied to the element A is reversed relative to that in vibration mode① , and, in the vibration mode③ , the phases of the driving voltages applied to the elements A and B are reversed. The driving voltage in each case is sinusoidal and has a frequency in the vicinity of the resonance frequency of the stator to which the vibration elements are attached.

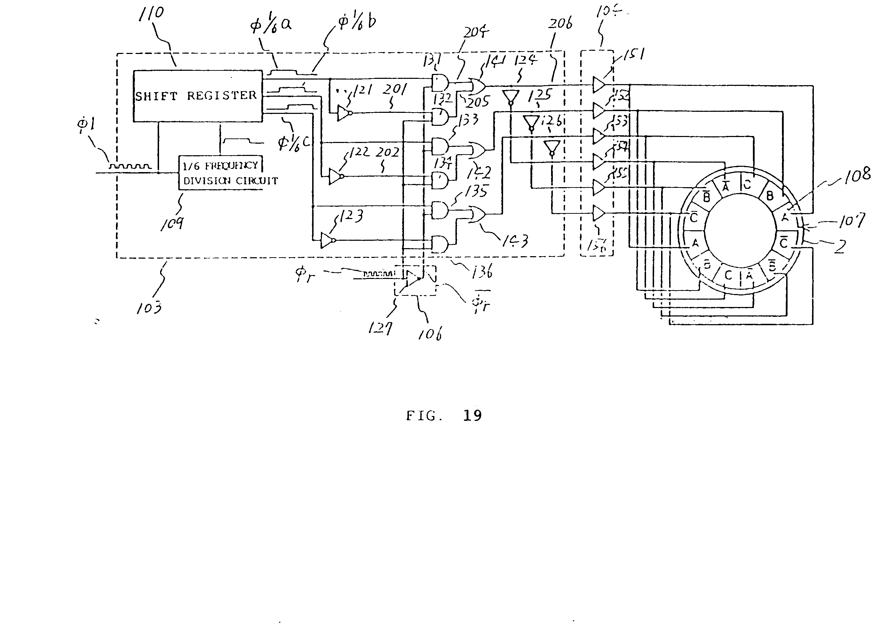

Figure 19 is a circuit diagram of the control circuit 103, the phase inversion circuit 106 and the driver circuit 104 of the drive circuit shown in figure 11 for use with the ultrasonic motor of figure 15 for example in which each rotation has 12 division steps and the rotory is driven 30° per step.

The control circuit 103 comprises a 1/6 frequency division circuit 109, a shift register 110, inverters 121, 122, 123, 124, 125 and 126, AND gates 131, 132, 133, 134, 135 and 136, and OR gates 141, 142, and 143. The phase inversion circuit 106 comprises an inverter 127. The driver circuit 104 comprises buffers 151, 152, 152, 154, 155 and 156. Positive and negative polarity voltages are supplied alternately to each respective buffer for outputing a driving voltage which vibrates between a positive and negative polarity. The driving voltage from each of the buffers 151, 152, 154, 155 and 156 is applied to one terminal of respective ones of the vibration elements 108, the other terminals being commonly grounded.

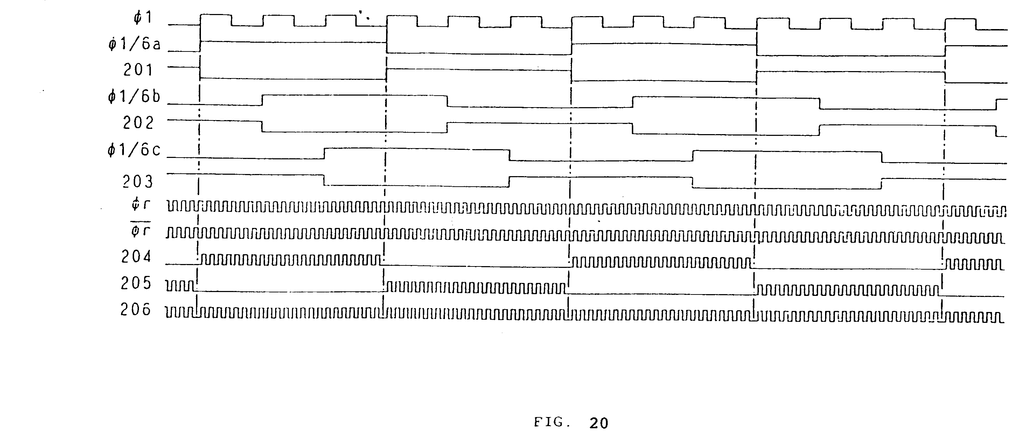

Figure 20 is a timing chart showing the signals of the drive circuit as described above. When a control signal Ø1 from the controller 102 is input to the frequency division circuit 109 and the shift register 110, a signal whose frequency is divided by 6 is output from the 1/6 frequency division circuit 109 and is supplied to the shift register 110. The shift register 110 outputs signals Ø1/6a, Ø1/6b and Ø1/6c, each of which is delayed by one pulse of Ø1 relative to the preceding signal and whose phase is reversed every three such pulses. The signal Ø1/6a is input to the AND gate 131. It is also inverted by the inverter 121 and the inverted signal 201 is input to the AND gate 132. Further, an oscillation signal Ør from the oscillation circuit 105, is supplied to the AND gate 132 and an inverted signal $\overline{Ø}r$ from the phase inversion circuit 106 is input to the AND gate 131. As a result, an output signal 204 formed from the logical AND of the signal Ø1/6a and the output signal $\overline{Ø}r$ is obtained from the AND gate 131. An output signal 205 formed from the logical AND of the inverted signal 201 and the oscillation signal $\overline{Ø}r$ is obtained from the AND gate 132. These output signals 204 and 205 are supplied to the buffer 151 via the OR gate 141 and are supplied to the buffer 154 by the inverter 124 as driving control signals for elements A and B.

The operation based on the signals Ø1/6b and Ø1/6c is basically the same as for the signal Ø1/6a. After inverted signals 202 and 203 are obtained for each one, they are processed in the same manner as for the above mentioned signal Ø1/6a. The phases of the driving control signals input to the buffers 152, 153, 155 and 156 are respectively delayed by 1 pulse in relation to each of the preceding driving control signals. Six driving voltages are thus output from the driver circuit 104, each buffer applying a driving voltage which oscillates at the frequency of the oscillation signal Ør between the positive and negative polarity to the vibration elements 108 on the basis of the received driving control signal. Two of the vibration elements 108 are driven at a certain moment. Therefore, three vibration elements 108 are driven in phase or at an opposite phase. Flexural vibration of wavelength 2 λ is generated, and the rotor 21 is driven stepwise by this oscillation.

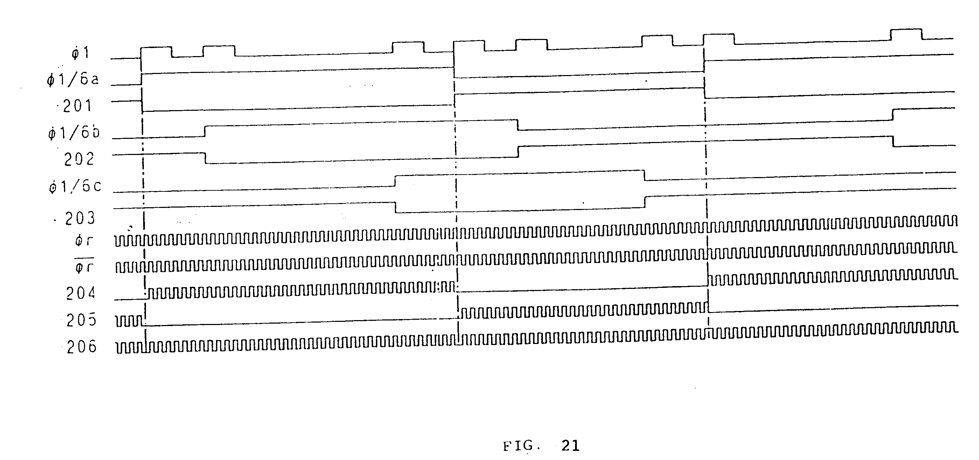

Figure 21 is a timing chart showing a modification in which the control signals Ø1 are irregular. As is obvious from this figure, since the control signal Ø1 prescribes the timing at which the phase of the voltage applied to each of the vibration elements 108 is switched, no problem is posed if the timing is irregular. For example, the discharge quantity of the apparatus of the present invention can easily be changed in dependence upon a time period, temperatures, the kinds of liquids discharged and the like by adjustment of the control signal Ø1 in each case.

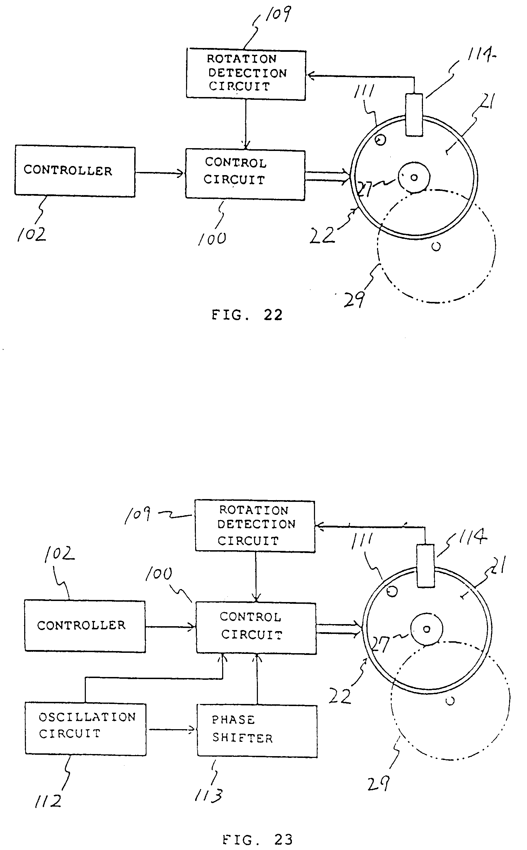

Figure 22 is a block diagram showing a modification of the drive circuit of figure 11 in which a motor using in a piezoelectric vibration element as disclosed in Japanese Laid-Open Patent Nos 61-54885, and 1-12882 is used as an electro-mechancial transducer in the discharge apparatus in the present invention. In this figure, feedback is employed in order to control the amount of the rotation of the motor. A rotation detection circuit 109 amplifies the output of a rotation detection element 114, which comprises, for example, a photo-interruptor of a like for detecting the passage of a mark 111 provided on the rotor 21, and supplies the amplified output to the control circuit 103. The control circuit 103 samples the output of the rotation detection circuit 109 until it reaches a number of rotations determined by the controller 102. During this time period, the control circuit 103 drives the piezoelectric element attached to the stator 22, and the rotational force of the motor is transmitted from the pinion 27 to the gear 29.

Figure 22 is a block diagram showing another modification of the drive circuit of figure 11 in which an ultrasonic motor of a travelling wave excitation type as disclosed in Japanese Laid-Open Patent Nos. 61-102177 and 63-181676 is used as an electro-mechanical transducer in the discharge apparatus of the present invention. The basic operation of this circuit is identical to that of the example shown in figure 22. However, an oscillation circuit 112 for generating a vibration signal for causing the stator to resonate and a phase shifter 113 for forming waveforms which are 90° out of phase are added for the purpose maintaining travelling wave excitation.

In the above disclosure, certain specific examples have been described. However, various modifications are possible within the scope of the invention.

For example, the form of the motor is not limited to that disclosed. Although in the above explanation, an example was given of 12-division step driving for one rotation realised by appropriately switching the phase of the driving voltage applied to each vibration element, this is not limiting. The ultrasonic stepping motor drives the motor stepwise as each vibration node moves, and therefore any motor at which at standing wave vibration may be provided can be employed. That is, the shapes of the rotor and the stator, the number of steps per rotation, the form of the vibration mode, the arrangement of the drive circuit and the like may be varied.

Since the ultrasonic stepping motor does not use magnetic force, no use of a magentic material is required. Therefore, the ultrasonic stepping motor does not give off eletro-magnetic noise, nor is it influenced by such noise from the outside.

This embodiment thus provides an apparatus which is not influenced by electro-magnetic noise, which does not provide magnetic influences to the outside, and which uses a motor employing a piezoelectric element having a high torque.

By using an ultrasonic stepping motor as a driving source, it is possible to manage with a small reduction ratio due to a high torque, and thus the apparatus can be miniaturised.

As explained above, the space (eg length) needed for the fluid container may be approximately half that required for a syringe of the piston and cylinder variety owing to the fact that bellows are provided in the storage section so that contraction is made possible.

Because the load at the time the bellows section is contracted or expanded can be limited and also fluctuations in the load can be limited, it is possible to manage with a stepping motor having a small torque, and thus the stepping motor need only consume a small amount of electric current. Therefore, a small electric battery can be used for a power supply, and further the life time of the electric battery can be lengthened.

As a result, a compact low-discharge apparatus can be made, and portability can be improved. In addition, it is possible to employ a thin, flat container for the discharge apparatus.

Furthermore, it is easy to control the discharge amount as a result of the use of a stepping motor.

## Claims

1. Fluid discharge apparatus comprising a container (3) for storing a fluid, means (4) for discharging fluid from the container, drive means (70) for operating the discharge means, and a drive circuit (40) for controlling the drive means, characterised in that the container (3) is contractible, and the discharge means (4) are arranged to engage a portion of the container and by the application of pressure to cause the container to contract for discharging the fluid.

2. The apparatus according to claim 1 characterised in that the container has a flexible wall (3f) whereby the container is contractible.

3. Apparatus according to claim 1 or 2 characterised in that the container has a bellows section (3f) whereby the container is contractible.

4. Apparatus according to any of claims 1 to 3 characterised in that the drive means (70) comprise an electro-mechanical transducer.

5. Apparatus according to claim 4 characterised in that the electro-mechanical transducer comprises a stepping motor having a rotor (72), a stator (73), and a coil block (74).

6. Apparatus according to claim 4 characterised in that the electro-mechanical transducer comprises an ultrasonic motor having a rotor (21), a stator (22), and a piezoelectric element (23).

7. Apparatus according to any preceding claim characterised in that the discharge means comprise an actuating lever (4), which engages directly or indirectly an end portion (3g) of the container.

8. Apparatus according to any preceding claim characterised in that the discharge means comprise a transmission mechanism for converting rotational motion of the drive means into linear motion for transmitting a driving force to said portion of the container.

9. Fluid discharge apparatus characterised by an electro-mechanical transducer (70), a circuit (40) for driving the electro-mechanical transducer, a power supply for operating the driving circuit, a container (3) whose section for storing a fluid is contractible, and a transmission mechanism (10) for transmitting the driving force of the electro-mechanical transducer to the container, the pressing of the container by a part of the transmission mechanism with the working of the circuit causing at least a part of the container to be contracted thereby causing the fluid to be discharged.

FIG. 1

FIG. 2

FIG. 3

FIG. 4

EP 0 465 267 A1

FIG. 5

FIG. 6

FIG. 7

80b

80

81a

81c

80d

81b

81c

80d

A

80a

80d

80a

80c

81c

80d

81

17

EP 0 465 267 A1

FIG. 8

FIG. 9

FIG. 10

EP 0 465 267 A1

FIG. 11

FIG. 12

FIG. 13

FIG. 14

FIG. 15

FIG. 16

FIG. 17

FIG. 18

FIG. 19

FIG. 20

EP 0 465 267 A1

FIG. 21

FIG. 22

FIG. 23

28

**European Patent Office**

# EUROPEAN SEARCH REPORT

Application Number

EP 91 30 6140

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | US-A-5 000 739 (KULISZ)<br>* Column 3, lines 34-68; figures 1,2 * | 1-5,9 | A 61 M 5/148 |
| A | | 7,8 | |
| | --- | | |
| X | US-A-3 884 228 (HAHN)<br>* Column 2, line 57 - column 5, line 22; column 7, lines 3-34; figures 1,2,12 * | 1-4,9 | |
| Y | | 5,6 | |
| A | | 7,8 | |
| | --- | | |
| X | DE-A-3 517 927 (B. BRAUN MELSUNGEN)<br>* Column 3, line 43 - column 5, line 14; figures 1-5 * | 1-4,9 | |
| A | | 7,8 | |
| | --- | | |
| X | US-A-4 929 234 (CHEN)<br>* Column 1, line 63 - column 3, line 28; figures 1-2 * | 1-4,9 | |
| A | | 7,8 | TECHNICAL FIELDS SEARCHED (Int. Cl.5) |
| Y | EP-A-0 376 497 (CYBOR)<br>* Column 2, line 39 - column 3, line 8; figure 1 * | 5 | A 61 M<br>F 04 B |
| A | | 1 | |
| | --- | | |
| Y | DE-A-3 810 660 (NIPPON ZEON CO.)<br>* Column 3, lines 10-62; figures 1,3 * | 6 | |
| A | | 1 | |
| | ----- | | |

The present search report has been drawn up for all claims

| Place of search | Date of completion of the search | Examiner |
|---|---|---|
| THE HAGUE | 26-09-1991 | BERTRAND G. |

EPO FORM 1503 03.82 (P0401)